# EUROPEAN PATENT SPECIFICATION

(11) **EP 3 556 415 B1**
(45) Date of publication and mention of the grant of the patent: **17.02.2021**
(21) Application number: 19160597.1
(22) Date of filing: 04.03.2019
(51) Int. Cl.: A61M 16/00, A61M 16/20

(54) **RESUSCITATION BAG WITH AUTOMATED VENTILATION CAPABILITIES**
REANIMATIONSTASCHE MIT AUTOMATISIERTEN BEATMUNGSFÄHIGKEITEN
SAC DE RÉANIMATION AVEC DES CAPACITÉS DE VENTILATION AUTOMATISÉES

(30) Priority: 20.04.2018 US 201862660291 P
(43) Date of publication of application: 23.10.2019
(73) Proprietor: Air Liquide Medical Systems, 92160 Antony (FR); L'AIR LIQUIDE, SOCIETE ANONYME POUR L'ETUDE ET L'EXPLOITATION DES PROCEDES GEORGES CLAUDE, 75007 Paris (FR)
(72) Inventor: BOULANGER, Thierry, Houston, Texas 77024 (US); RICHARD, Jean-Christophe, 92182 ANTONY (FR)
(74) Representative: Air Liquide

(56) References cited:
- EP-A1- 3 488 890
- EP-B1- 2 343 097
- WO-A1-00/20061

## Description

### Background

The present invention relates to an artificial respiration device, namely a resuscitation bag system with automated ventilation capabilities that can be used for resuscitating a person, i.e. a patient, in state of cardiac arrest.

For treating a person in cardiac arrest, it is known to realize a cardiac massage comprising thoracic compressions or 'TCs' along with intervals of lung ventilation with a resuscitation bag system.

TCs comprise successive compressions manually operated by a rescuer on the thoracic cage of the person, i.e. the patient, in cardiac arrest so that its thoracic cage goes "down" and CO₂-containing gases are expelled out of the lungs. Two successive TCs are separated by a decompression or release phase, during which the rescuer stops exerting a manual pressure on the thorax of the patient so that the thoracic cage can goes "up" again, and fresh O₂-containing gases can enter into the lungs. TCs aim at partially restoring inspiration and expiration phases and therefore gas exchanges in the lungs, as well as promoting or restoring a blood circulation toward and in the brain of the patient.

As the successive compression/decompression phases only mobilize small volumes of gas in and out of the patient's airways, it is advocated to regularly perform additional gas insufflations to bring an oxygen-containing gas to the lungs, thereby enhancing the gas exchanges, especially the removal of CO₂ from the blood and the entering of O₂.

Fresh O₂-containing gas can be delivered to the patient by means of a resuscitation bag system associated to an oxygen source, such as a gas cylinder containing medical-grade O₂, that is fluidly connected to the patient via a respiratory interface, typically a facial mask, a laryngeal mask or an endotracheal tube.

It is currently recommended to interpose insufflations of gas about every 15 TCs, knowing that the ideal rate of compressions is of between 100 and 120 compressions per minute (c/min) - therefore 8 to 10 insufflations per minute, according to international guidelines.

As multiple studies have shown that interrupting TCs for an appropriate duration can significantly impact the patient's condition, it is not possible for only one rescuer to realize continuous TCs at a TC rate of between 100 and 120c/min, while at a same time, performing gas insufflations about every 15 TCs. In practice, two rescuers are therefore mobilized and must cooperate synchronously: when the first rescuer (in charge of the thoracic compressions) stops the TCs, the second (in charge of the ventilation) starts the periodic insufflations, and vice versa.

However, requiring two rescuers is not ideal either as they are fully-occupied with the TCs and insufflations, and hence are not available for further treating the patient, for instance for injecting him/her with drugs. WO00/20061 describes an automated cardiopulmonary resuscitation device.

### Summary

One goal of the present invention is to propose an artificial respiration device, namely a resuscitation bag system useable for resuscitating a person, i.e. a patient, in state of cardiac arrest that requires only one rescuer for performing the TCs and gas insufflations.

More specifically, the present invention discloses an improved resuscitation bag system comprising automated ventilation capabilities so that it is able to recognize the status of the TCs and to periodically perform automatic insufflations.

A solution concerns a resuscitation bag system useable for resuscitating, i.e. for insufflating, a person in state of cardiac arrest, comprising:
- a deformable bag comprising a gas inlet and a gas outlet,
- a gas conduit in fluid communication with the gas outlet of the deformable bag,
- a pneumatic control valve comprising an exhaust port for controlling the flow of gas exiting to the atmosphere through said exhaust port,
- a monitoring module arranged on the pneumatic control valve,
- an insufflation module electrically connected to the monitoring module and fluidly connected to the enclosure, and
- an enclosure defining a hollow volume,
and wherein:
- the flexible bag is arranged into the hollow volume of the enclosure, and
- the insufflation module is operated by the monitoring module for controlling a gas delivery into the enclosure.

A resuscitation bag system comprises one or several of the following additional features:
- the insufflation module is fluidly connected to a source of gas, typically an oxygen cylinder.
- the insufflation module further comprises a first one-way valve arranged in the gas conduit between the gas outlet of the deformable bag, and the pneumatic control valve.
- the insufflation module further comprises a derivation conduit having:
   i) a first end fluidly connected to the gas conduit, between the gas outlet of the deformable bag and the first one-way valve, and
   ii) a second end fluidly connected to the pneumatic control valve.
- the monitoring module comprises at least one sensor.
- said at least one sensor is configured for measuring at least one parameter of the gas and transmitting at least one signal corresponding to said at least one parameter to the insufflation module.
- said at least one sensor comprises a pressure sensor.
- the insufflation module delivers gas into the enclosure in response to at least one pressure signal transmitted by the monitoring module.

Described is a method for resuscitating a person, i.e. a patient, in state of cardiac arrest, comprising providing gas insufflations to said person by means of a resuscitation bag system according to the present invention.

Preferably, the gas comprises air or oxygen-enriched air.

### Brief Description of the Drawings

The present invention will be explained in more details in the following illustrative description of an embodiment of a resuscitation bag system useable for insufflating a person in state of cardiac arrest, in the frame of a resuscitation provided by a rescuer, which is made in references to the accompanying drawings among them:
- Figures 1-3 show a resuscitation bag system,
- Figure 4 shows a monitoring module for the resuscitation bag system of Figures 1-3,
- Figure 5 shows an example of a pressure oscillation curve resulting from successive TCs operated on a patient,
- Figures 6-10 illustrate an embodiment of an enclosure for a flexible bag of a resuscitation bag system according to the present invention,
- Figure 11 illustrates an embodiment of a resuscitation bag system according to the present invention,
- Figure 12 shows an embodiment of a monitoring module for the resuscitation bag system of Figure 11,
- Figure 13 shows an embodiment of an insufflation module for the resuscitation bag system of Figure 11,
- Figure 14 represents the successive steps operated by an algorithm run by the monitoring module of a resuscitation bag system according to the present invention, and
- Figures 15 to 20 show the way the resuscitation bag system of Figure 11 works.

### Description of Preferred Embodiments

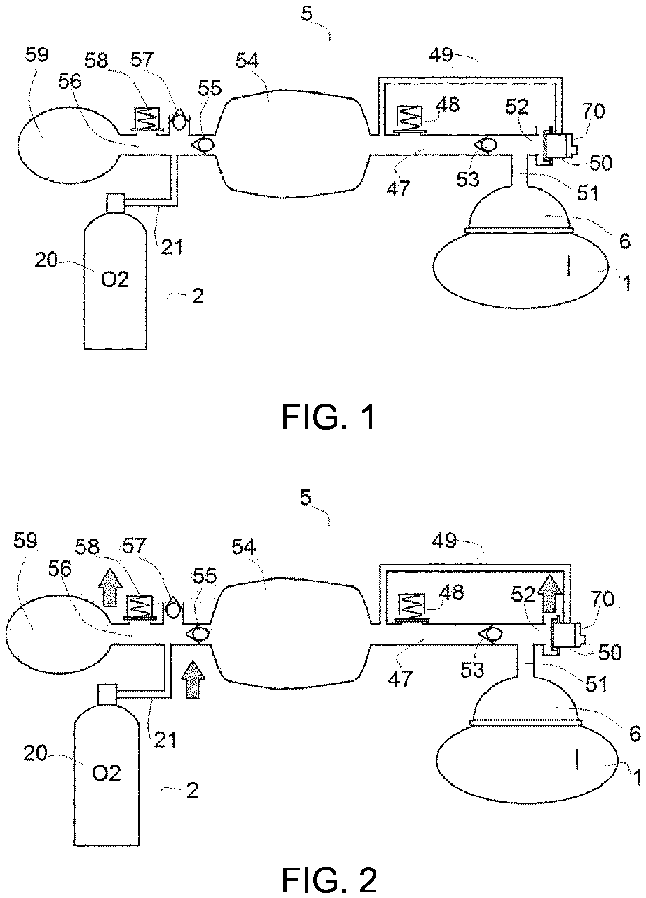

Figure 1 shows an embodiment of a resuscitation bag **5** system comprising a respiratory interface **6** for feeding a gas to a patient **1,** typically a respiratory mask, a hollow flexible bag **54,** a valve element **50,** also called control valve **50,** for diverting the gas in and out of a patient **1,** during insufflation and exsufflation phases, a monitoring module **70** connected to said valve element **50** and a source of an oxygen-containing gas **2,** such as a or including a gas cylinder **20** containing oxygen or an oxygen-containing gas.

The O₂-containing gas contained in the flexible bag **54** is provided to the respiratory interface **6,** when said flexible bag **54** is squeezed by a rescuer, via tubing elements **47, 51** that ensure a fluid communication between the flexible bag **54** and the respiratory interface **6.** A one-way valve **53** arranged in tubing element **47** controls the gaseous flow travelling in said tubing element **47** so that the gas can only travel from flexible bag **54** to interface **6.**

Further, a derivation conduit **49** is arranged between tubing element **47** and the valve element **50** for controlling the opening or closing of the valve element **50.**

Such a resuscitation bag **5** system allows performing a gas insufflation to the patient **1,** when a rescuer is squeezing the flexible bag **54,** i.e. a deformable enclosure, while the patient receives continuous thoracic compressions (TCs) operated by another rescuer.

Continuous TCs, without pausing for a gas insufflation step, are made possible by controlling valve element **50** which opens whenever the pressure gradient existing between conduit **51** and derivation conduit **49** exceeds a given opening pressure, i.e. a given pressure threshold.

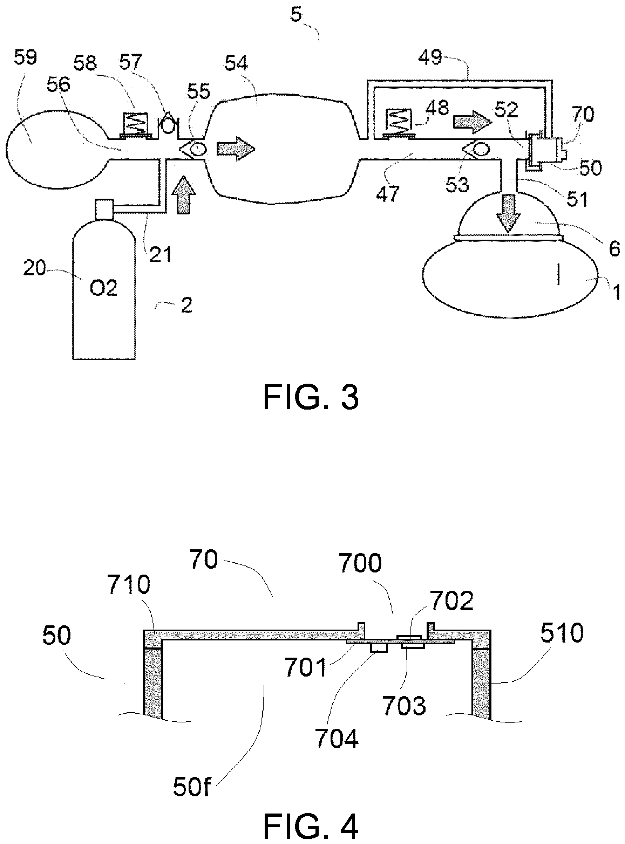

The resuscitation bag system **5** further comprises a monitoring module **70** comprising a module enclosure **710** that is connected to the valve enclosure **510** of the valve element **50,** as shown in Figure 4. The monitoring module **70** exhibits an electronic board **700** comprising a control unit **702,** such as a microcontroller, a power source **703,** such as a coin cell battery, and a pressure sensor **704.** These components are laid out on a board **701,** securely attached to the module enclosure **710.** Typically, board **701** comprises electronic communications between the different components **702, 703, 704** so as to power said components and allow the transmission of data from pressure sensor **704** to control unit **702** for further processing.

As can be seen, the pressure sensor **704** is located in chamber **50f** of the valve element **50,** thereby allowing a monitoring of the pressure in said valve **50** element for providing to the rescuer pressure values representative of the pressure existing in the patient's **1** airways.

Figures 2 and 3 show TCs performed by a rescuer with the resuscitation bag system **5** at rest, e.g. without any action on the flexible bag **54.**

During a thoracic compression, as shown in Figure 2, the positive pressure that is generated by the TCs, closes the one-way valve **53** and the gaseous flow coming out of the lungs of the patient **1** and recovered into interface **6,** further travels successively in tubing elements **51, 52,** i.e. gas conduits, passages or the like, and is afterwards vented to the atmosphere through valve element **50,** with the proviso that the positive pressure thus created is greater than the opening pressure of valve element **50.**

As a consequence, the monitoring module **70** monitors a pressure increase at the patient's airways that depends on the opening pressure of valve element **50** and a flow resistance of said valve element **50,** i.e. the flow of gas expelled by patient **1.**

Similarly, Figure 3 shows what happens during a decompression or release phase following a TC, i.e. during the release phase between two successive TCs.

After the termination of a TC, i.e. when the rescuer stops pushing on the patient's chest, the pressure in the patient's airways suddenly decreases to potentially sub-atmospheric pressures. As a consequence, the flow of oxygen coming from tubing **21** and travelling in first conduit element **56,** is directed to the patient 1 to offset the pressure decrease, thereby opening the second one-way valve **55** and the first one-way valve **53,** and allowing the gas flow to circulate through flexible bag **54** and tubing elements **47, 51** to the interface **6.**

In addition, the pressure across valve element **50** equals to about 0 and as a result the valve element **50** is closed. This pressure decrease in the patient airways is captured by the monitoring module **70.**

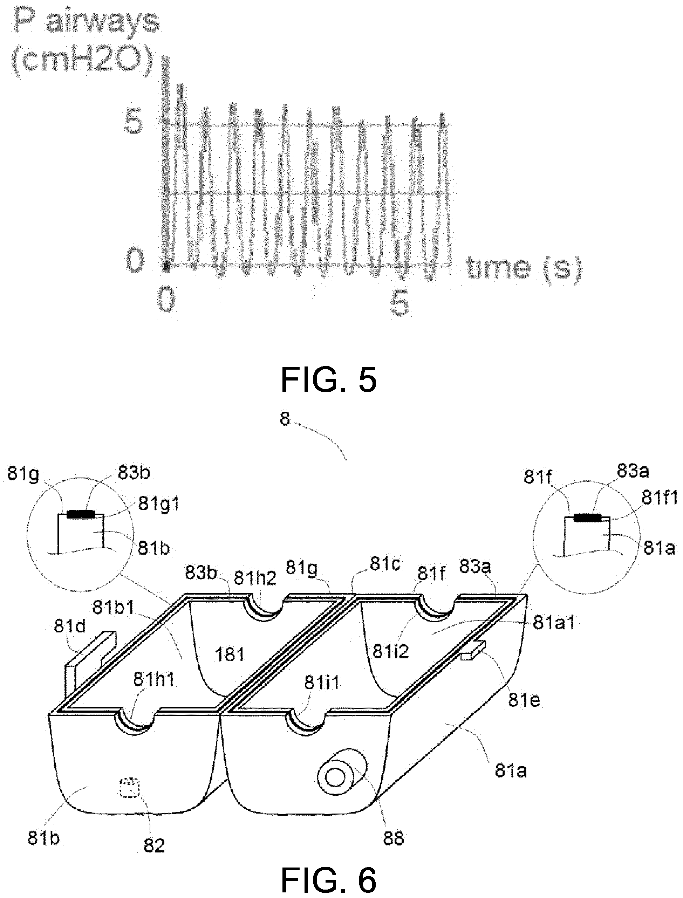

Successive TCs generate pressure oscillations that the monitoring module **70** is able to record and process. An example of a pressure oscillation curve is shown in Figure 5. Extracting the frequency from said oscillations can be easily done by processing algorithms.

However, with a resuscitation bag system **5** as shown in Figures 1-3, two rescuers are required, i.e., one for performing the TCs and another one for performing the gas insufflations.

In the frame of the present invention, the resuscitation bag system **5** shown in Figures 1-3 has been modified so that only one rescuer is required for doing both, i.e. the TCs and the gas insufflations.

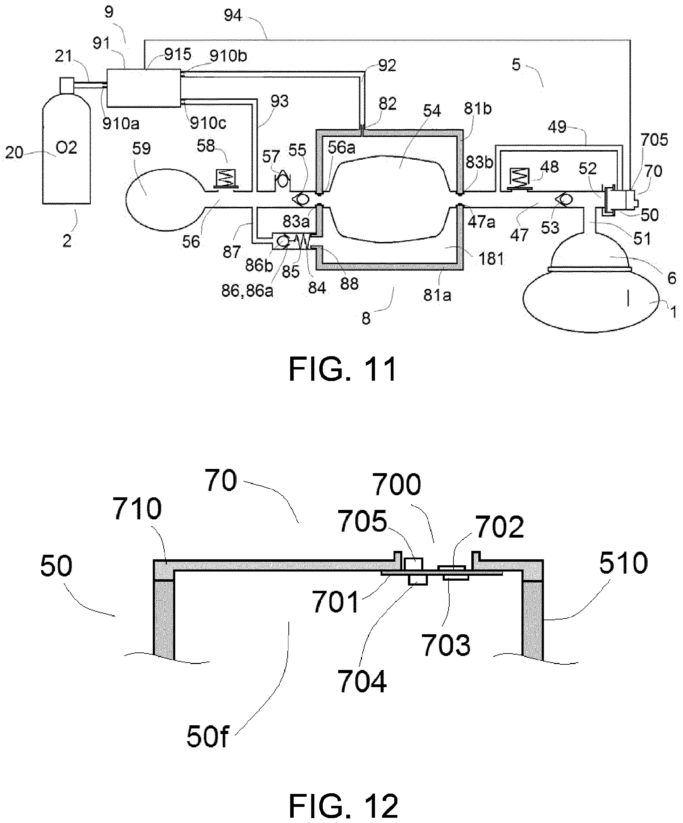

An embodiment of a resuscitation bag system **5** according to the present invention is shown in Figure 11, which incorporates a hollow enclosure **8** forming a hollow chamber **181** for receiving the flexible bag **54** of the resuscitation bag system **5,** as detailed hereafter.

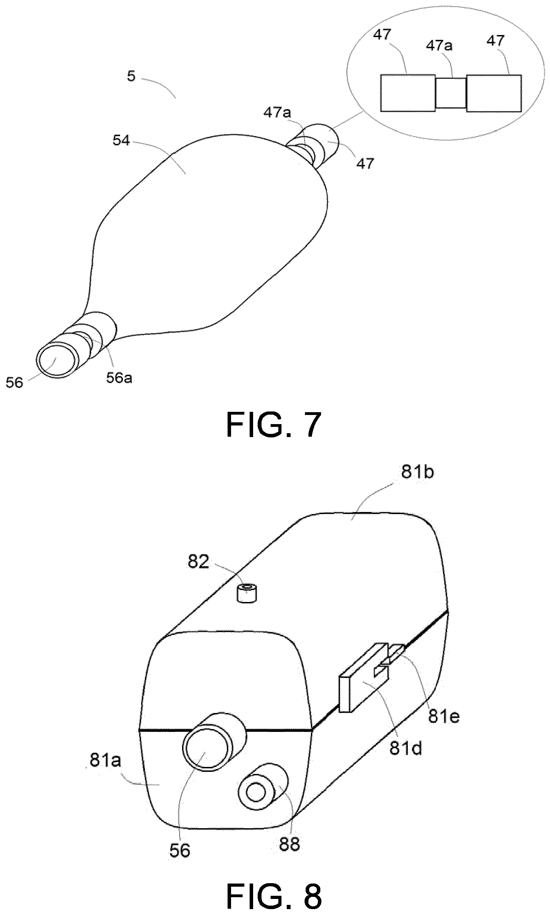

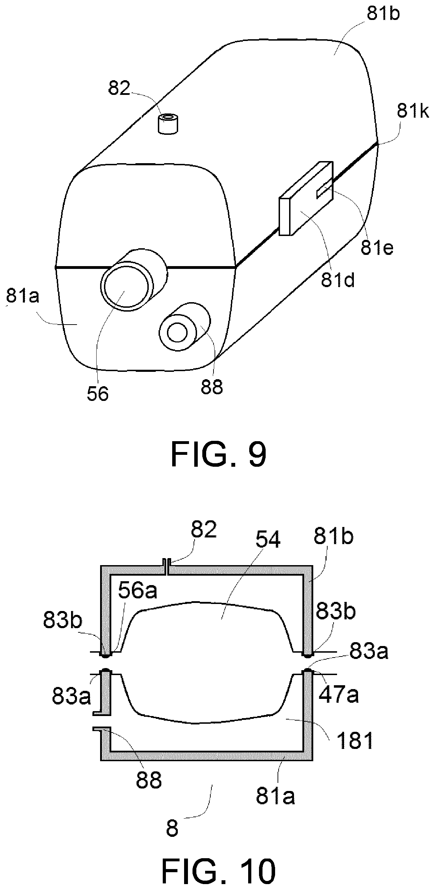

Figure 6 illustrates an embodiment of an enclosure **8** for a resuscitation bag system **5** according to the present invention, shown in an open position, whereas Figures 8 and 9 show the enclosure **8,** once closed.

In the present embodiment, the enclosure **8** is made of two main parts cooperating together, comprising a lower cover **81a** and upper cover **81b,** i.e. two half-shells, that can be assembled and fixed together for defining an inner hollow volume **81a1, 81b1,** called chamber **181,** for receiving the flexible bag **54** of the resuscitation bag system **5** as detailed hereafter.

The upper and lower covers **81a, 81b** are preferably made of a rigid material, such as plastic material, e.g. ABS, or any other suitable material.

The upper and lower covers **81a, 81b** are attached together by a pivoting hinge **81c,** such as a thin strip of plastic material, so that the upper cover **81b** can pivot around the hinge **81c** to come into contact with lower cover **81a** thereby closing the enclosure **8** and defining the inner hollow volume, i.e. chamber **181,** including the inner volumes **81a1, 81b1** of the lower and upper covers **81a, 81b.**

The lower cover **81a** further exhibits arc-shaped apertures **81i1, 81i2,** whereas the upper cover **81b** presents corresponding arc-shaped apertures **81h1, 81h2,** whose arc-shaped apertures **81i1, 81i2, 81h1, 81h2** defining passages for the tubular ends of the bag **54,** as shown in Figure 7, when said flexible bag **54** is positioned into inner hollow volume of the enclosure **8,** i.e. when the flexible bag **54** is sandwiched between the upper and lower covers **81a, 81b.**

Peripheral grooves **81f1, 81g1** are arranged in the peripheral borders or edges **81f,** 81g of lower and upper covers **81a, 81b,** and annular gaskets **83a, 83b** are arranged therein. Gaskets **83a, 83b** are made of the same material, typically silicon or the like.

The lower cover **81a** further comprises a lower port **88** in fluid communication with the inner volume **81a1** of the lower cover **81a.** In other words, the lower port **88** forms a gas passage traversing the peripheral wall of the lower cover **81a.** Similarly, the upper cover **81b** also comprises an upper port **82** in fluid communication with the inner volume **81b1** of the upper cover **81b.** Here again, the upper port **82** forms a gas passage traversing the peripheral wall of the upper cover **81b.**

Figure 7 is a 3D representation of a flexible insufflation bag **54** of a resuscitation bag system **5** that is designed and configured for being inserted into hollow chamber **181** of enclosure **8** of Figure 6.

Said flexible insufflation bag **54** comprises an inlet conduit **56** and outlet conduit **47** at its opposite ends. Inlet conduit **56** and outlet conduit **47** both comprise annular grooves **56a, 47a** arranged in their outer peripheral walls. Annular grooves **56a, 47a** are designed so as to fit with arc-shaped apertures, **81i1, 81h1, 81i2, 81h2** arranged in the upper and lower covers **81a, 81b** forming enclosure **8,** when insufflation bag **54** is sandwiched between said upper and lower covers **81a, 81b,** as shown in Figures 8 and 9. In other words, insufflation bag **54** is held in position inside the enclosure **8** thanks to the annular grooves **56a, 47a** of the inlet and outlet conduits **56, 47** of insufflation bag **54** that cooperate with the peripheral wall of the enclosure **8** in the areas of the arc-shaped apertures, **81i1, 81h1, 81i2, 81h2.**

Figures 8 and 9 show the flexible insufflation bag **54** of the resuscitation bag system **5** placed and hold in enclosure **8** with the upper cover **81b** pivoted so as to be in mechanical contact with the lower cover **81a.**

As one can see, the upper cover **81b** further comprises a sliding locker **81d,** whereas the lower cover **81a** comprises a protruding element **81e.** The sliding locker **81d** and the protruding element **81e** cooperate together so as to form a locking mechanism that can be used for maintaining the upper cover **81b** integral with the lower cover **81a.** Of course, such locking mechanism is only illustrative and other locking systems can be adopted.

In Figure 8 the protruding element **81e** is not held by the sliding locker **81d** (i.e. unlocked position), whereas in Figure 9 the protruding element **81e** is held by the sliding locker **81d,** i.e. the protruding element **81e** is inserted into a lodging, such as a slot, arranged in the sliding locker **81d** thereby prohibiting any pivoting of the upper cover **81b** (i.e. locked position).

In the locked position, the lower and upper covers **81a, 81b** are firmly held together, whereas the gaskets **83a, 83b** are firmly compressed one against the other for ensuring a tight seal **81k** between lower cover **81a,** upper cover **81b** and the insufflation bag **54** of the resuscitation bag system **5.** The insufflation bag **54** is then comprised in a seal-tight volume as shown in Figure 10.

Figure 11 is a cross sectional illustration of an embodiment of the entire resuscitation bag system **5** according to the present invention.

The architecture of the resuscitation bag system **5** of Figure 11 is similar to the one of Figure 1, but comprises additional features according to the present invention.

In particular, the resuscitation bag system **5** of Figure 11 comprises the enclosure **8** of Figures 6, to 8 and 9, as well as further modifications such as the annular grooves **56a, 47a** of inlet and outlet conduits **47, 56** (Fig. 7) to realize a tight connection with enclosure **8** as described hereabove. The resuscitation bag **5** exhibits additional lines or tubings **87, 93** in fluidic connection with the inlet conduit **56.** Tubing **87** is fluidly connected to a chamber **84,** itself fluidly connected to port **88** of enclosure **8.** Chamber **84** includes an overpressure valve system **86, 86a** comprising a ball **86b,** typically a metallic ball, resting on a cup **86a.** The ball **86b** is integral with a spring **85** which exerts a force on ball **86b** and force it to stay in cup **86a,** preventing any fluidic connection between hollow chamber **181** and tubing **87.** However, the spring **85** develops a maximum force which therefore defines an opening pressure. Should the pressure in hollow chamber **181** exceeds the opening pressure, the spring **85** expands and the ball **86b** moves away from cup **86a,** making a fluidic connection between hollow chamber **181** and tubing **87.** The opening pressure can be of various value and can be set, as an example, to about 100 cm H₂O. The role of this overpressure valve system is explained below.

The monitoring module **70** of resuscitation bag system **5** further comprises an electrical connector **705,** laid out on a board **701** and electronically connected to control unit **702** and power source **703** for controlling an external module.

An external module of resuscitation bag system **5** of Figure 11 is an insufflation module **9,** exhibiting an external casing **91** comprising several ports including:
- a first pneumatic port **910a** fluidly connected to tubing **21** connected to the oxygen source **20,** typically an oxygen cylinder,
- a second pneumatic port **910b** fluidly connected to tubing **92,** itself connected to the port **82** of enclosure **8,**
- a third pneumatic port **910c** fluidly connected to tubing **93,** and
- an electrical connector **915** electrically connecting the insufflation module **9** to the monitoring module **70,** via an electrical cable **94** and the electrical connector **705** of the monitoring module **70.**

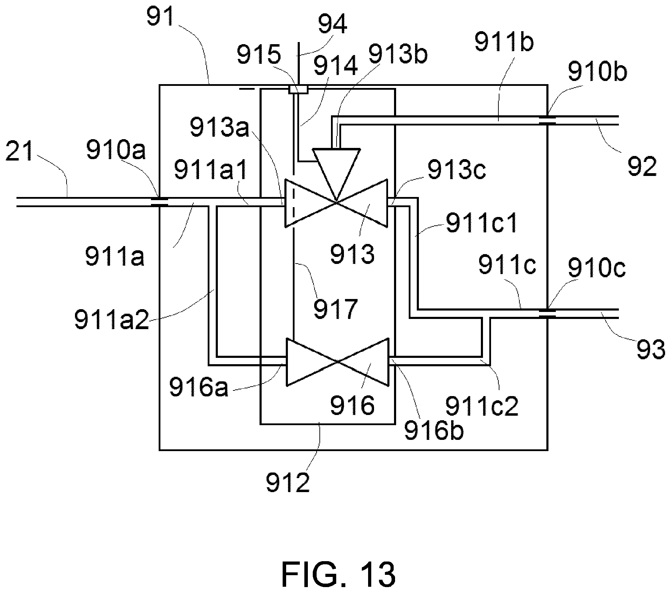

Figure 13 shows the internal architecture of the insufflation module 9. It comprises an electronic board **912** which connects via electric wire **914** electrical connector **915** to a 3:2 valve **913.** Such 3:2 valve has the ability to fluidly connecting two different ports together. As an example, at rest, the 3:2 way valve **913** makes a fluidic connection between ports **913b** and **913c.** As port **913b** is connected to tubing **911b,** itself connected to port **910b** and tubing **92,** and port **913c** is connected to tubing **911c1,** itself further prolonged by tubing **911c,** connected to port **910c** and tubing **93,** a fluidic connection is performed between said tubing **92** and tubing **93.**

Conversely, when 3:2 valve **913** is actuated, ports **913a** and **913b** are in fluidic connection. As port **913b** is connected to tubing **911b,** itself connected to port **910b** and tubing **92,** and port **913a** is connected to tubing **911a1** itself further prolonged by tubing **911a,** connected to port **910a** and tubing **21,** a fluidic connection is performed between said tubings **21** and **92.** Actuation of 3:2 valve **913** is performed through the electrical connection existing between monitoring module **70** and insufflation module **9** via electrical wire **94** connected on one side to the monitoring module **70** via connector **705** and on the other side to connector **915,** further electrically connected to 3:2 valve **913** via electrical wire **914.**

The electronic board **912** further connects via electric wire **917** electrical connector **915** to a 2:2 valve **916.** Such 2:2 valve has the ability to be opened, e.g. performs a fluidic connection between its ports **916a** and **916b** or closed (i.e., rest position), preventing any fluidic connection between port **916a** and port **916b.**

When 2:2 valve is actuated, port **916a** and port **916b** are in fluidic connection. As port **916a** is connected to tubing **911a2,** itself further prolonged by tubing **911a,** connected to port **910a** and tubing **21,** and port **916b** is connected to tubing **911c2** itself further prolonged by tubing **911c,** connected to port **910c** and tubing **93,** a fluidic connection is performed between said tubings **21, 93.**

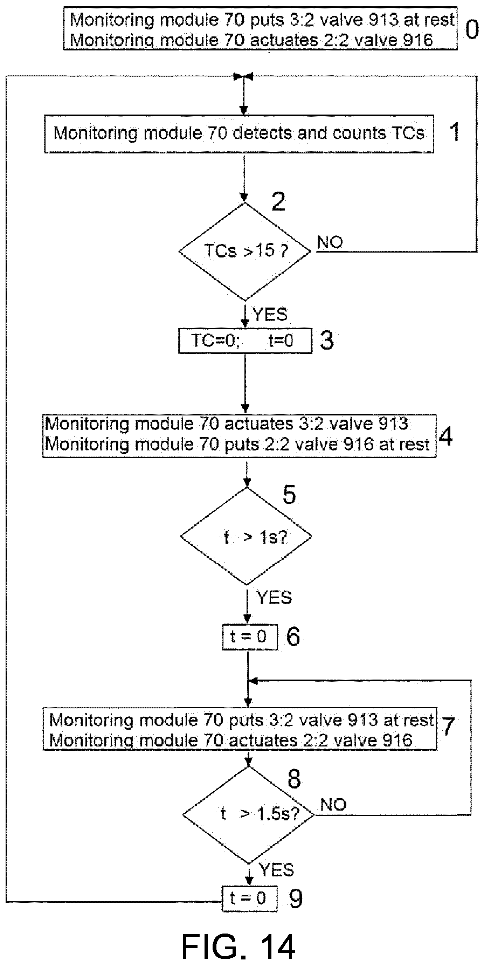

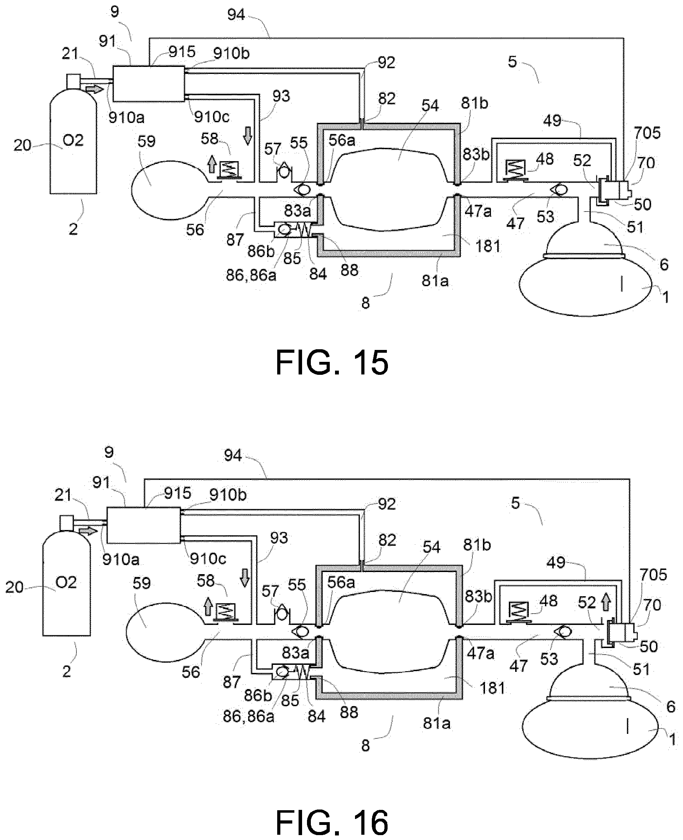

The operation mode of the entire resuscitation bag system **5,** in particular the relationships between flexible bag **54,** enclosure **8** and insufflation module **9,** is illustrated in Figures 15 to 20, whereas Figure 14 represents the successive steps operated by an algorithm run by the monitoring module **70** (control unit **702**) of the resuscitation bag system **5** according to the present invention.

Those successive steps of Figure 14 are as follows:

### Step 0

As shown in Figure 15, the monitoring module **70** of the resuscitation bag system **5** controls the insufflation module **9** via electric wire **94** and puts 3:2 valve **913** at rest and actuates 2:2 valve **916.** Consequently, tubing **21** is fluidly connected to tubing **93,** whereas tubing **93** is itself fluidly connected to tubing **92.** Oxygen coming from the O₂ source **20** travels into tubings **21, 93** before entering into conduit **56** and filling the reserve **59,** i.e. a gas reservoir. A pressure equilibrium is reached in the elements downstream of one-way valve **55,** such as bag **54** and conduits **47, 49** as well as in the airways of the patient **1,** via conduits **51, 52** and interface **6.** In this initial phase, the control valve **50** is closed due to the pressure equilibrium.

Simultaneously, a pressure equilibrium is reached between tubings **93, 92** and therefore chamber **181** of enclosure **8** as tubing **92** is connected to port **82.** The pressure inside the chamber is limited to the maximum pressure defined by the opening pressure of a PEEP exhaust valve **58,** typically about 5 cm H₂O which is not enough for overcoming the force developed by spring **85** and therefore keeps the ball **86b** on its cup **86a,** preventing any fluidic connection of chamber **181** and tubing **87.** After the pressure equilibrium is reached and reserve **59** is filled, the oxygen in excess is vented through PEEP exhaust valve **58.**

### Step 1

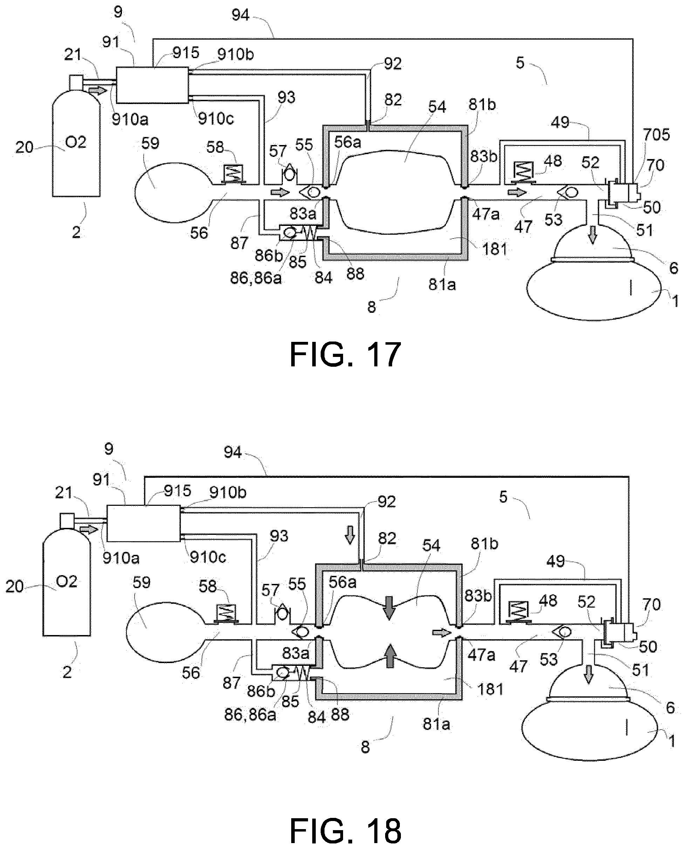

A sequence of TCs is performed by a rescuer on a patient in cardiac arrest as illustrated in Figures 16 and 17.

During a TC (Fig. 16), the positive pressure which is generated by the gases going out of the patient's lungs, closes one-way valve **53** as the flow travels into interface **6,** conduits **51, 52** to be vented to the atmosphere through control valve **50,** when the positive pressure is greater than the opening pressure of said control valve **50.**

As a consequence, the monitoring module **70** detects a pressure increase at patient's airways. This pressure increase depends on the opening pressure of control valve **50** and resistance to flow of said control valve **50,** i.e. flow expelled by patient 1.

Similarly, a TC is followed by a decompression phase (cf. Fig. 17). The pressure in the patient's airways suddenly decreases to potentially sub-atmospheric pressures, i.e. < 1 atm. As a consequence, the flow of oxygen coming from tubing **93** and reserve **59** is directed via conduit **56** to the patient 1 to offset this decrease in pressure, opening the one-way valves **55, 53,** and travelling through flexible bag **54,** conduits **47, 51** and interface **6.** In addition, the pressure across the control valve **50** is close to 0 and as a result, said control valve **50** is closed. Said pressure decrease at the patient's airways is also captured by the monitoring module **70.**

As already said, TCs create pressure oscillations (Fig. 5) that the monitoring module **70** can record and process, in particular for counting the number of TCs.

### Step 2

Once the number of TCs is considered to be greater than a given value, for instance **15** in the present example (cf. Fig. 14), or any other suitable value predefined by the rescuer, the monitoring initializes the insufflation sequence.

### Step 3

Refreshing of the variables such as the time t and number of TCs, that are all set to an initial value of 0 (cf. Fig. 14).

### Step 4

The monitoring module **70** then commands the insufflation module **9** via electric wire **94** by actuating the 3:2 valve **913** and putting the 2:2 valve **916** at rest. In other words, a fluidic connection is operated between the oxygen source **20** and tubing **21** to tubing **92** further connected to port **82** and therefore chamber **181** of enclosure **8** as shown in Fig. 18. As the 2:2 valve **916** is at rest, the tubing **93** is no longer fluidly connected to the oxygen source **20** via tubing **21** and therefore no oxygen does flow into conduit **56.**

As the flexible bag **54** is tightly sealed in enclosure **8,** a pressure buildup is generated in chamber **181** and a pneumatic force is applied on flexible bag **54** thereby squeezing said flexible bag **54** and expelling gas therefrom. This mimics a manual compression normally done by a second rescuer.

The volume of gas expelled from flexible bag **54** opens the one-way valve **53** located downstream of the flexible bag 54. The gas pressure across control valve **50** is then close to 0 and, as a result, control valve **50** remains closed. The flow of gas exiting the bag **54** travels into conduits **47, 51** and is delivered to the patient **1,** via interface **6.**

Upstream of the flexible bag **54,** such positive pressure forces the one-way valve **55** to remain closed and therefore the upstream part of the resuscitation bag system **5,** especially PEEP exhaust valve **58** and reserve **59** are at "rest" since no oxygen is delivered by tubing **93.** This means that reserve **59** remains (at least partially) filled of gas, whereas PEEP exhaust valve **58** is closed.

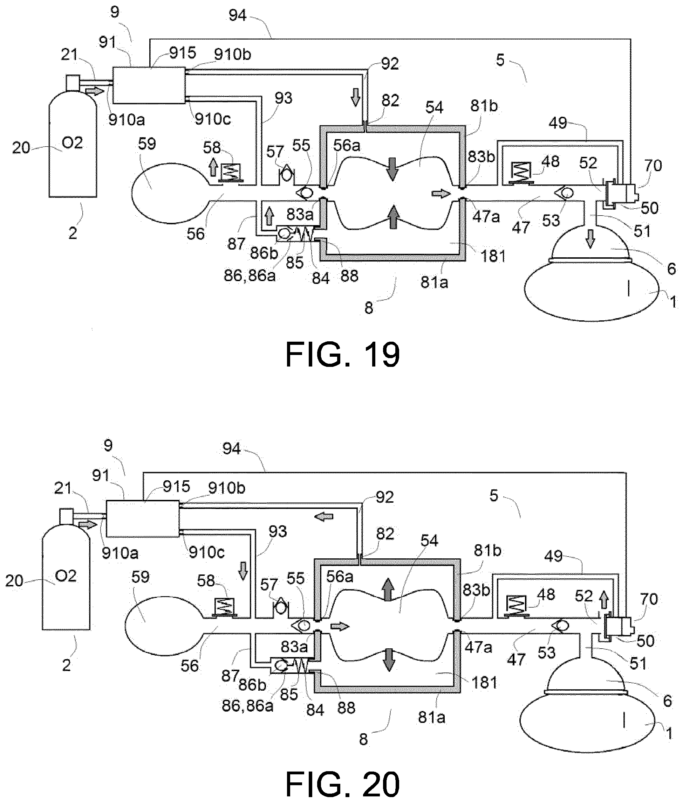

As shown in Figure 19, ball **86b** moves away from its cup **86a** whenever the pressure into chamber **181** exceeds the spring load **85,** set as example to 100 cm H₂O, thereby avoiding any excessive pressure buildup.

This creates a fluidic connection between chamber **181** and tubing **87** so that gas in excess is vented into conduit **56** and refill the reserve **59,** if needed, or escapes through PEEP exhaust valve **58** whenever the pressure into conduit **56** exceeds the opening pressure of PEEP exhaust valve **58,** e.g. 5 cm H₂O for example.

### Step 5

The monitoring module **70** of resuscitation bag system **5** which includes control unit **702** computes the time that has elapsed.

As soon the insufflation time is greater than 1s (or any other time set by the rescuer depending on their protocol), the monitoring module **70** switches to the expiration phase.

### Step 6

The monitoring module **70** resets the time t to 0.

### Step 7

During an expiration phase, the monitoring module **70** controls the insufflation module **9,** via electric wire **94,** and puts 3:2 valve **913** at rest and further actuates 2:2 valve **916.** Consequently, tubing **21** is fluidly connected to tubing **93** and tubing **93** is fluidly connected to tubing **92** (cf. Fig. 20).

As the pressure within chamber **181** is greater than the pressure in conduit **56,** a backward flow travels between chamber **181** and conduit **56,** via tubings **92** and **93.** Said backward flow dramatically decreases the pressure in chamber **181** so that the ball **86b** applies on cup **86a** due to the spring **85** so as to prevent any fluid communication with tubing **87.**

The flexible bag **54** can then enter into an expansion phase thereby creating a negative pressure differential across one-way valve **55.** This negative pressure differential allows opening one-way valve **55** and letting the gas flowing into conduit **56** (via tubing **93**) enter the flexible bag **54** to fill it up. The gas circulating into tubing **93** is mainly composed of the backward flow coming from chamber **181** via tubing **92.** Further, an additional flow coming from the oxygen source **20** and tubing **21** also circulates into tubing **93.** This additional flow helps making sure that the PEEP exhaust valve **58** is open (e.g. pressure in conduit **56** is positive, e.g. of 5 cm H₂O).

Downstream of the flexible bag **54,** the negative pressure differential holds back one-way valve **53** and decreases the pressure in derivation conduit **49** and then in control valve **50.** As the pressure in the patient's airways is high, as a result from the past insufflation, the control valve **50** opens allowing the volume expired by the patient **1** to travel through interface **6,** conduits **51** and **52** and be vented to the atmosphere.

The control valve **50** remains open until an equilibrium is reached between pressures in conduits **47, 51,** i.e. around the pressure set by the PEEP exhaust valve **58,** e.g. about 5 cm H₂O. A low pressure level applies then into the patient's lungs.

### Step 8

A fixed time for the expiration phase is set and the monitoring module starts another sequence whenever the expiration time exceeds 1.5 sec (or any other duration preset by the rescuer).

### Step 9

An initialization of time t is then done.

More generally speaking, a resuscitation bag system **5** according to the present invention comprises automated ventilation capabilities, e.g. being able to recognize the status of the TCs, count them and perform periodically automatic insufflations, so that only one rescuer is needed for operating the TCs to a patient in cardiac arrest, whereas the gas insufflations are automatically-operated based on the monitoring of TCs (for example one insufflation is performed every 15 TCs). It should be obvious that such resuscitation bag system **5** would likewise be able to dispense an insufflation based on time so as to provide, for example 8 to 10 breaths/minute.

## Claims

1. A resuscitation bag system (5) useable for resuscitating a person (1) in state of cardiac arrest, comprising:
- a deformable bag (54) comprising a gas inlet and a gas outlet,
- a gas conduit (47) in fluid communication with the gas outlet of the deformable bag (54), and a first conduit element (56) in fluid communication with the gas inlet of the deformable bag (54)
- a pneumatic control valve (50) comprising an exhaust port for controlling the flow of gas exiting to the atmosphere through said exhaust port,
- a monitoring module (70) arranged on the pneumatic control valve (50),
- an insufflation module (9) electrically connected to the monitoring module (70) and fluidly connected to an enclosure (8), and
- the enclosure (8) defining a hollow volume (181), and wherein:
- the flexible bag (54) is arranged into the hollow volume (181) of the enclosure (8), and
- the insufflation module (9) is operated by the monitoring module (70) for controlling a gas delivery into the enclosure (8),
**characterized in that** the insufflation module (9):
i) is fluidly connected to an oxygen source (2) providing oxygen to said insufflation module (9),
ii) is further fluidly connected to the first conduit element (56) for providing oxygen to said conduit element (56), and
iii) is configured for delivering oxygen gas into the enclosure (8) in response to at least one pressure signal transmitted by the monitoring module (70).

2. The resuscitation bag system according to claim 1, wherein the source of oxygen (2) is an oxygen cylinder.

3. The resuscitation bag system according to claim 1, wherein it further comprises a first one-way valve (53) arranged in the gas conduit (47) between the gas outlet of the deformable bag (54), and the pneumatic control valve (50).

4. The resuscitation bag system according to claim 1, wherein it further comprises a derivation conduit (49) having:
i) a first end fluidly connected to the gas conduit (47), between the gas outlet (54B) of the deformable bag (54) and the first one-way valve (53), and
ii) a second end fluidly connected to the pneumatic control valve (50).

5. The resuscitation bag system according to claim 1, wherein the monitoring module (70) comprises at least one sensor.

6. The resuscitation bag system according to claim 5, wherein said at least one sensor is configured for measuring at least one parameter of the gas and transmitting at least one signal corresponding to said at least one parameter to the insufflation module (9).

7. The resuscitation bag system according to claim 6, wherein said at least one sensor comprises a pressure sensor.

8. The resuscitation bag system according to claim 1, wherein the insufflation module (9) comprises an external casing (91) comprising :
- a first pneumatic port (910a) fluidly connected to a first tubing (21), said first tubing (21) being fluidly connected to an oxygen cylinder (20),
- a second pneumatic port (910b) fluidly connected to a second tubing (92), said second tubing (92) being fluidly connected to a port (82) of the enclosure (8), and
- a third pneumatic port (910c) fluidly connected to a third tubing (93), said third tubing (93) being fluidly connected to conduit element (56).

9. The resuscitation bag system according to claim 8, wherein the external casing (91) of the insufflation module (9) further comprises a first electrical connector (915) for electrically connecting the insufflation module (9) to a second electrical connector (705) of the monitoring module (70).

## Patentansprüche

1. Reanimationsbeutelsystem (5), das zur Reanimation einer Person (1) bei einem Herzstillstand verwendbar ist, umfassend:
- einen verformbaren Beutel (54), der einen Gaseinlass und einen Gasauslass umfasst,
- eine Gasleitung (47) in Fluidkommunikation mit dem Gasauslass des verformbaren Beutels (54) und ein erstes Leitungselement (56) in Fluidkommunikation mit dem Gaseinlass des verformbaren Beutels (54)
- ein pneumatisches Steuerventil (50), das eine Auslassöffnung zur Steuerung des Durchsatzes von Gas umfasst, das durch die Auslassöffnung in die Atmosphäre austritt,
- ein Überwachungsmodul (70), das auf dem pneumatischen Steuerventil (50) angeordnet ist,
- ein Einblasmodul (9), das elektrisch mit dem Überwachungsmodul (70) verbunden ist und fluidisch mit einem Gefäß (8) verbunden ist, und
- wobei das Gefäß (8) ein Hohlvolumen (181) definiert,
und wobei:
- der flexible Beutel (54) in das Hohlvolumen (181) des Gefäßes (8) angeordnet wird, und
- das Einblasmodul (9) von dem Überwachungsmodul (70) betrieben wird, um eine Gaszufuhr in das Gefäß (8) zu steuern,
**dadurch gekennzeichnet, dass** das Einblasmodul (9):
i) fluidisch mit einer Sauerstoffquelle (2) verbunden ist, die Sauerstoff für das Einblasmodul (9) bereitstellt,
ii) weiter fluidisch mit dem ersten Leitungselement (56) verbunden ist, um dem Leitungselement (56) Sauerstoff bereitzustellen, und
iii) dazu konfiguriert ist, als Reaktion auf mindestens ein vom Überwachungsmodul (70) gesendetes Drucksignal Sauerstoffgas in das Gefäß (8) zuzuführen.

2. Reanimationsbeutelsystem nach Anspruch 1, wobei die Sauerstoffquelle (2) eine Sauerstoffflasche ist.

3. Reanimationsbeutelsystem nach Anspruch 1, wobei es weiter ein erstes Einwegventil (53) umfasst, das in der Gasleitung (47) zwischen dem Gasauslass des verformbaren Beutels (54) und dem pneumatischen Steuerventil (50) angeordnet ist.

4. Reanimationsbeutelsystem nach Anspruch 1, wobei es weiter eine Ableitungsleitung (49) umfasst, die Folgendes aufweist:
i) ein erstes Ende, das zwischen dem Gasauslass (54B) des verformbaren Beutels (54) und dem ersten Einwegventil (53) fluidisch mit der Gasleitung (47) verbunden ist, und
ii) ein zweites Ende, das fluidisch mit dem pneumatischen Steuerventil (50) verbunden ist.

5. Reanimationsbeutelsystem nach Anspruch 1, wobei das Überwachungsmodul (70) mindestens einen Sensor umfasst.

6. Reanimationsbeutelsystem nach Anspruch 5, wobei der mindestens eine Sensor dazu konfiguriert ist, mindestens einen Parameter des Gases zu messen und mindestens ein dem mindestens einen Parameter entsprechendes Signal an das Einblasmodul (9) zu senden.

7. Reanimationsbeutelsystem nach Anspruch 6, wobei der mindestens eine Sensor einen Drucksensor umfasst.

8. Reanimationsbeutelsystem nach Anspruch 1, wobei das Einblasmodul (9) ein externes Gehäuse (91) umfasst, umfassend:
- eine erste pneumatische Öffnung (910a), die fluidisch mit einer ersten Rohrleitung (21) verbunden ist, wobei die erste Rohrleitung (21) fluidisch mit einer Sauerstoffflasche (20) verbunden ist,
- eine zweite pneumatische Öffnung (910b), die fluidisch mit einer zweiten Rohrleitung (92) verbunden ist, wobei die zweite Rohrleitung (92) fluidisch mit einer Öffnung (82) des Gefäßes (8) verbunden ist, und
- eine dritte pneumatische Öffnung (910c), die fluidisch mit einer dritten Rohrleitung (93) verbunden ist, wobei die dritte Rohrleitung (93) fluidisch mit einem Leitungselement (56) verbunden ist.

9. Reanimationsbeutelsystem nach Anspruch 8, wobei das externe Gehäuse (91) des Einblasmoduls (9) weiter einen ersten elektrischen Stutzen (915) zum elektrischen Verbinden des Einblasmoduls (9) mit einem zweiten elektrischen Stutzen (705) des Überwachungsmoduls (70) umfasst.

## Revendications

1. Système de sac de réanimation (5) utilisable pour la réanimation d'une personne (1) en état d'arrêt cardiaque, comprenant :
- un sac déformable (54) comprenant une entrée de gaz et une sortie de gaz,
- un conduit de gaz (47) en communication fluidique avec la sortie de gaz du sac déformable (54), et un premier élément de conduit (56) en communication fluidique avec l'entrée de gaz du sac déformable (54)
- une soupape de commande pneumatique (50) comprenant un port d'évacuation pour la commande de l'écoulement du gaz sortant vers l'atmosphère à travers ledit port d'évacuation,
- un module de surveillance (70) agencé sur la soupape de commande pneumatique (50),
- un module d'insufflation (9) connecté électriquement au module de surveillance (70) et connecté fluidiquement à une enceinte (8), et
- l'enceinte (8) définissant un volume creux (181), et dans lequel :
- le sac souple (54) est agencé dans le volume creux (181) de l'enceinte (8), et
- le module d'insufflation (9) est actionné par le module de surveillance (70) pour la commande d'une distribution de gaz dans l'enceinte (8),
**caractérisé en ce que** le module d'insufflation (9) :
i) est connecté fluidiquement à une source d'oxygène (2) fournissant de l'oxygène audit module d'insufflation (9),
ii) est en outre connecté fluidiquement au premier élément de conduit (56) pour la fourniture d'oxygène audit élément de conduit (56), et
iii) est configuré pour la distribution de gaz d'oxygène dans l'enceinte (8) en réponse à au moins un signal de pression transmis par le module de surveillance (70).

2. Système de sac de réanimation selon la revendication 1, dans lequel la source d'oxygène (2) est un cylindre d'oxygène.

3. Système de sac de réanimation selon la revendication 1, dans lequel il comprend en outre une première soupape de non-retour (53) agencée dans le conduit de gaz (47) entre la sortie de gaz du sac déformable (54), et la soupape de commande pneumatique (50).

4. Système de sac de réanimation selon la revendication 1, dans lequel il comprend en outre un conduit de dérivation (49) présentant :
i) une première extrémité connectée fluidiquement au conduit de gaz (47), entre la sortie de gaz (54B) du sac déformable (54) et la première soupape de non-retour (53), et
ii) une seconde extrémité connectée fluidiquement à la soupape de commande pneumatique (50).

5. Système de sac de réanimation selon la revendication 1, dans lequel le module de surveillance (70) comprend au moins un capteur.

6. Système de sac de réanimation selon la revendication 5, dans lequel ledit au moins un capteur est configuré pour mesurer au moins un paramètre du gaz et transmettre au moins un signal correspondant audit au moins un paramètre au module d'insufflation (9).

7. Système de sac de réanimation selon la revendication 6, dans lequel ledit au moins un capteur comprend un capteur de pression.

8. Système de sac de réanimation selon la revendication 1, dans lequel le module d'insufflation (9) comprend un boîtier externe (91) comprenant :
- un premier port pneumatique (910a) connecté fluidiquement à une première tubulure (21), ladite première tubulure (21) étant connectée fluidiquement à un cylindre d'oxygène (20),
- un deuxième port pneumatique (910b) connecté fluidiquement à une deuxième tubulure (92), ladite deuxième tubulure (92) étant connectée fluidiquement à un port (82) de l'enceinte (8), et
- un troisième port pneumatique (910c) connecté fluidiquement à une troisième tubulure (93), ladite troisième tubulure (93) étant connectée fluidiquement à l'élément de conduit (56).

9. Système de sac de réanimation selon la revendication 8, dans lequel le boîtier externe (91) du module d'insufflation (9) comprend en outre un premier connecteur électrique (915) pour la connexion électrique du module d'insufflation (9) à un second connecteur électrique (705) du module de surveillance (70).
